# EUROPEAN PATENT SPECIFICATION

(11) **EP 2 822 495 B1**
(45) Date of publication and mention of the grant of the patent: **04.04.2018**
(21) Application number: 13757413.3
(22) Date of filing: 28.02.2013
(51) Int. Cl.: A61B 18/12, A61B 17/03

(54) **VESSEL SEALING INSTRUMENT**
GEFÄSSVERSIEGELUNGSVORRICHTUNG
INSTRUMENT D'ISOLEMENT DE VAISSEAUX

(30) Priority: 08.03.2012 US 201213415471
(43) Date of publication of application: 14.01.2015
(60) Divisional application: 16163749.1
(73) Proprietor: Covidien LP, Mansfield, MA 02048 (US)
(72) Inventor: ARTALE, Ryan C., Boulder, Colorado 80305 (US); MOUA, Tony, Broomfield, Colorado 80020 (US); SHARP, Robert M., Boulder, Colorado 80304 (US)
(74) Representative: Soames, Candida Jane
(86) International application number: PCT/US2013/028286
(87) International publication number: WO 2013/134044

(56) References cited:
- EP-A1- 1 532 932
- CN-A- 101 507 635
- US-A- 6 010 516
- US-A1- 2004 092 927
- US-A1- 2007 106 297
- US-A1- 2008 215 048
- US-A1- 2008 319 442
- US-A1- 2009 240 246
- US-A1- 2010 130 977
- US-A1- 2011 009 859
- US-B2- 7 744 615

## Description

### BACKGROUND

### 1_{.} Background of Related Art

The present disclosure relates to forceps used for open surgical procedures. More particularly, the present disclosure relates to a forceps that applies electrosurgical current to seal tissue.

### 2. Technical Field

A hemostat or forceps is a simple plier-like tool which uses mechanical action between its jaws to constrict vessels and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue.

Certain surgical procedures require sealing and cutting blood vessels or vascular tissue, Several journal articles have disclosed methods for sealing small blood vessels using electrosurgery. An article entitled Studies on Coagulation and the Development of an Automatic Computerized Bipolar Coagulator, J. Neurosurg., Volume 75, July 1991, describes a bipolar coagulator which is used to seal small blood vessels. The article states that it is not possible to safely coagulate arteries with a diameter larger than 2 to 2.5 mm. A second article is entitled Automatically Controlled Bipolar Electrocoagulation - "COA-COMP", Neurosurg. Rev. (1984), pp. 187-190, describes a method for terminating electrosurgical power to the vessel so that charring of the vessel walls can be avoided.

By utilizing an electrosurgical forceps, a surgeon can either cauterize, coagulate/desiccate, reduce or slow bleeding and/or seal vessels by controlling the intensity, frequency and duration of the electrosurgical energy applied to the tissue. Generally, the electrical configuration of electrosurgical forceps can be categorized in two classifications: 1) monopolar electrosurgical forceps; and 2) bipolar electrosurgical forceps.

Monopolar forceps utilize one active electrode associated with the clamping end effector and a remote patient return electrode or pad which is typically attached externally to the patient. When the electrosurgical energy is applied, the energy travels from the active electrode, to the surgical site, through the patient and to the return electrode.

Bipolar electrosurgical forceps utilize two generally opposing electrodes which are disposed on the inner opposing surfaces of the end effectors and which are both electrically coupled to an electrosurgical generator. Each electrode is charged to a different electric potential. Since tissue is a conductor of electrical energy, when the effectors are utilized to grasp tissue therebetween, the electrical energy can be selectively transferred through the tissue.

In order to effect a proper seal with larger vessels, two predominant mechanical parameters must be accurately controlled - the pressure applied to the vessel and the gap between the electrodes both of which affect thickness of the sealed vessel. More particularly, accurate application of the pressure is important to oppose the walls of the vessel, to reduce the tissue impedance to a low enough value that allows enough electrosurgical energy through the tissue, to overcome the forces of expansion during tissue heating and to contribute to the end tissue thickness which is an indication of a good seal. It has been determined that a fused vessel wall is optimum between 0.001 and 0.006 inches. Below this range, the seal may shred or tear and above this range the lumens may not be properly or effectively sealed.

With respect to smaller vessel, the pressure applied to the tissue tends to become less relevant whereas the gap distance between the electrically conductive surfaces becomes more significant for effective sealing. In other words, the chances of the two electrically conductive surfaces touching during activation increases as the vessels become smaller.

Electrosurgical methods may be able to seal larger vessels using an appropriate electrosurgical power curve, coupled with an instrument capable of applying a large closure force to the vessel walls. It is thought that the process of coagulating small vessels is fundamentally different than electrosurgical vessel sealing. For the purposes herein, "coagulation" is defined as a process of desiccating tissue wherein the tissue cells are ruptured and dried and vessel sealing is defined as the process of liquefying the collagen in the tissue so that it reforms into a fused mass. Thus, coagulation of small vessels is sufficient to permanently close them. Larger vessels need to be sealed to assure permanent closure.

Numerous bipolar electrosurgical forceps have been proposed in the past for various open surgical procedures. However, some of these designs may not provide uniformly reproducible pressure to the blood vessel and may result in an ineffective or non-uniform seal. For example, U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stern et al., U.S. Patent No. 5,484,436 to Eggers et al. and U.S. Patent No. 5,951,549 to Richardson et al., as well as US2009/240246 all relate to electrosurgical instruments for coagulating, cutting and/or sealing vessels or tissue.

Many of these instruments include blade members or shearing members which simply cut tissue in a mechanical and/or electromechanical manner and are relatively ineffective for vessel sealing purposes. Other instruments rely on clamping pressure alone to procure proper sealing thickness and are not designed to take into account gap tolerances and/or parallelism and flatness requirements which are parameters which, if properly controlled, can assure a consistent and effective tissue seal. For example, it is known that it is difficult to adequately control thickness of the resulting sealed tissue by controlling clamping pressure alone for either of two reasons: 1) if too much force is applied, there is a possibility that the two poles will touch and energy will not be transferred through the tissue resulting in an ineffective seal; or 2) if too low a force is applied, a thicker less reliable seal is created.

### SUMMARY

The invention is defined by the electrosurgical instrument of independent claim 1. Preferred embodiments are defined by the dependent claims. Any disclosed methods are merely exemplary and do not fall within the scope of the present invention. Any example or embodiment which does not fall under the scope of the claims is not part of the invention. According to one aspect of the present disclosure, a bipolar electrosurgical instrument is provided. The instrument includes a housing and an elongated shaft extending from the housing. An end effector is coupled to a distal end of the elongated shaft. A handle assembly is operably coupled to the housing and includes a movable handle movable relative to a fixed handle for effecting movement of the jaw members relative to one another from a first position wherein the jaw members are disposed in spaced relation relative to one another to a second position wherein the jaw members cooperate to grasp tissue therebetween. Each jaw member is configured to connect to a source of electrosurgical energy such that the jaw members are configured to selectively conduct energy through tissue held therebetween to effect a tissue seal. A switch is disposed on the fixed handle and is configured to be depressed between a first position and at least one subsequent position upon biasing engagement with a switch engaging surface disposed on the movable handle, The first position of the switch causes the relay of information to the user corresponding to a desired pressure on tissue grasped between the jaw members and the at least one subsequent position is configured to activate the source of electrosurgical energy to supply electrosurgical energy to the jaw members.

Alternatively or in addition, a knife channel may be defined along a length of at least one of the jaw members and the electrosurgical instrument may include a cutting mechanism configured to reciprocate along the knife channel to cut tissue grasped between the jaw members.

Alternatively or in addition, the bipolar electrosurgical instrument may include an actuator for selectively advancing the cutting mechanism from a first position wherein the cutting mechanism is disposed proximal to tissue grasped between the jaw members to at least one subsequent position wherein the cutting mechanism is disposed distal to tissue grasped between the jaw members.

Alternatively or in addition, the switch may generate a first tactile response upon movement thereof to the first position and a subsequent tactile response upon movement thereof to the at least one subsequent position,

Alternatively or in addition, the desired pressure range may be measured by at least one strain gauge disposed within the forceps.

Alternatively or in addition, the first position of the switch may correspond to an initial closure pressure of the moveable handle relative to the fixed handle and the at least one subsequent position of the switch corresponds to a subsequent closure pressure of the movable handle relative to the fixed handle that is greater than the initial closure pressure.

Alternatively or in addition, the bipolar electrosurgical instrument may include a safety lockout configured to prevent reciprocation of the cutting mechanism when the jaw members are disposed in the first position.

Alternatively or in addition, each of the jaw members may include an electrically conductive sealing surface and at least one of the jaw members may include at least one non-conductive stop member disposed on the electrically conductive sealing surface configured to control the distance between opposing electrically conductive sealing surfaces when tissue is held therebetween.

According to a further aspect of the present disclosure, a bipolar electrosurgical instrument is provided. The instrument includes a housing and an elongated shaft extending from the housing. An end effector is coupled to a distal end of the elongated shaft. A handle assembly is operably coupled to the housing and includes a movable handle movable relative to a fixed handle for effecting movement of the jaw members relative to one another from a first position wherein the jaw members are disposed in spaced relation relative to one another to a second position wherein the jaw members cooperate to grasp tissue therebetween. Each jaw member is configured to connect to a source of electrosurgical energy such that the jaw members selectively conduct energy through tissue held therebetween to effect a tissue seal. A knife channel is defined along a length of at least one of the jaw members. A cutting mechanism is configured to reciprocate along the knife channel to cut tissue grasped between the jaw members. An actuator selectively advances the cutting mechanism from a first position wherein the cutting mechanism is disposed proximal to tissue grasped between the jaw members to at least one subsequent position wherein the cutting mechanism is disposed distal to tissue grasped between the jaw members. A switch is disposed on the fixed handle and is configured to be depressed between a first position and at least one subsequent position upon biasing engagement with a switch engaging surface disposed on the movable handle. The first position of the switch causes the relay of information to the user corresponding to a desired pressure on tissue grasped between the jaw members and the at least one subsequent position is configured to activate the source of electrosurgical energy to supply electrosurgical energy to the jaw members.

Alternatively or in addition, the first position of the switch may correspond to an initial closure pressure of the first and second shafts and the at least one subsequent position of the switch may correspond to a subsequent closure pressure of the first and second shafts that is greater than the initial closure pressure.

Alternatively or in addition, the bipolar electrosurgical instrument may include a safety lockout configured to prevent reciprocation of the cutting mechanism when the jaw members are disposed in the first position.

According to a further aspect of the present disclosure, a method of performing an electrosurgical procedure is provided. The method includes the step of moving a first handle relative to a second handle of a bipolar forceps to grasp tissue between first and second jaw members. The method also includes the step of depressing a switch upon movement of the first shaft relative to the second shaft to a first position to relay information to a user corresponding to a predetermined grasping pressure applied to tissue grasped between the jaw members. The method also includes the step of depressing the switch to at least one subsequent position to activate a source of electrosurgical energy to supply electrosurgical energy to the jaw members.

The method may also include the step of selectively advancing a cutting mechanism from a first position wherein the cutting mechanism is disposed proximal to tissue grasped between the jaw members to at least one subsequent position wherein the cutting mechanism is disposed distal to tissue held between the jaw members.

The method may also include the step of generating a first tactile response corresponding to the first position of the switch.

The method may also include the step of generating a second tactile response corresponding to the at least one subsequent position of the switch.

In the drawings and in the description that follows, the term "proximal", as is traditional, will refer to the end of electrosurgical instrument that is closer to the user, while the term "distal" will refer to the end that is further from the user.

### BRIEF DESCRIPTION OF THE DRAWINGS

Various embodiments of the subject instrument are described herein with reference to the drawings wherein:
Fig. 1 is a right, perspective view of a forceps according to one embodiment of the present disclosure;
Fig. 2 is an exploded view of the forceps of Fig. 1;
Fig. 3A is an exploded view of an end effector assembly of the forceps of Fig. 1;
Fig. 3B is a cross-sectional view of the end effector assembly of the forceps of Fig. 1;
Fig. 4A is a side view of the forceps of Fig. 1 with parts partially removed to show the electrical connection between a switch and the end effector assembly;
Fig. 4B is a left, perspective view of a jaw member of the end effector assembly of Fig. 1;
Fig. 4C is a left, perspective view of a jaw member of the end effector assembly of Fig. 1; and
Figs. 5A-5C are side views of the forceps of Fig. 1 illustrating actuation thereof between open and closed positions;
Fig. 6 is a side view of a knife for use with the forceps of Fig. 1 according to one embodiment of the present disclosure; and
Figs. 7A and 7B are side views of forceps according to one embodiment of the present disclosure.

### DETAILED DESCRIPTION

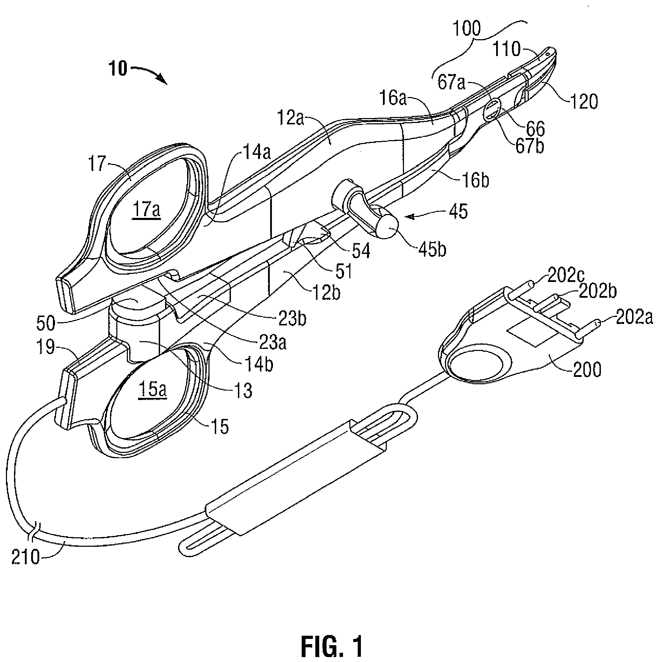

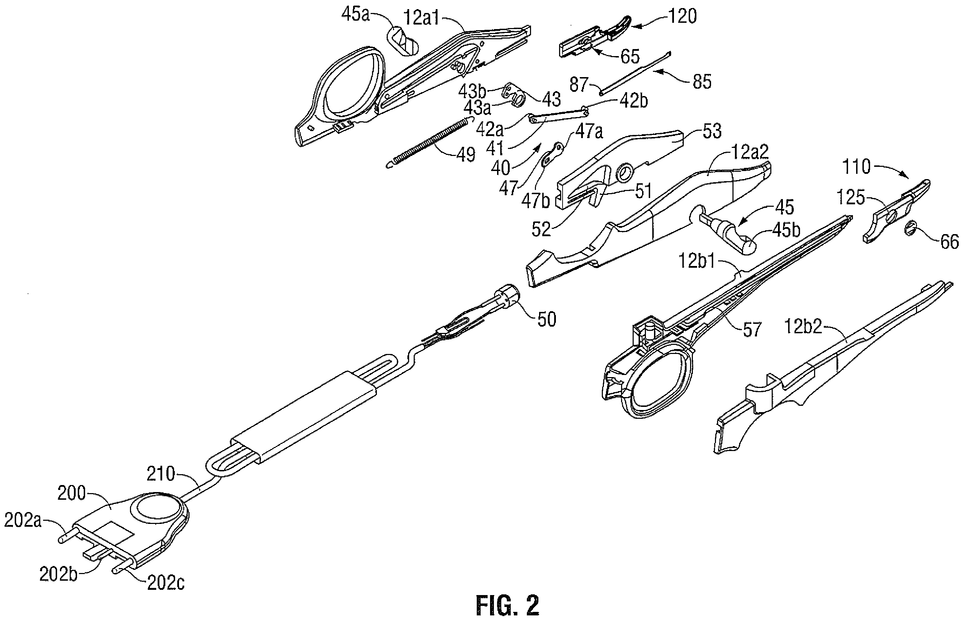

Referring initially to Figs. 1 and 2, a forceps 10 for use with open surgical procedures includes elongated shaft portions 12a and 12b each having a proximal end 14a, 14b and a distal end 16a and 16b, respectively.

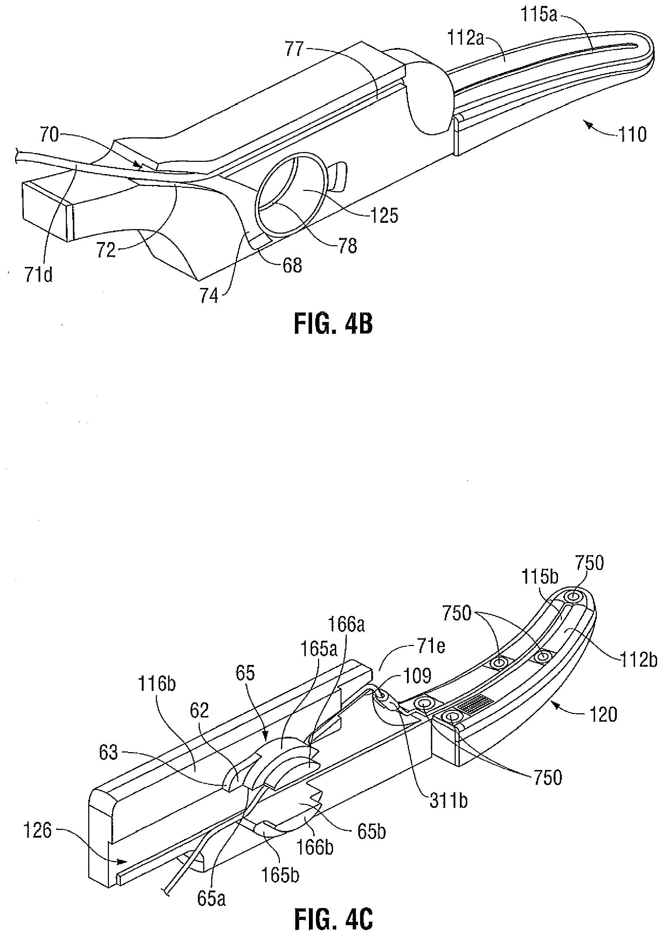

The forceps 10 includes an end effector assembly 100 that attaches to the distal ends 16a and 16b of shafts 12a and 12b, respectively. The end effector assembly 100 includes pair of opposing jaw members 110 and 120 that are pivotably connected and movable relative to one another about a pivot 65 (Fig. 2) to grasp tissue. Pivot 65 is disposed on a proximal end of jaw member 120 and includes opposing halves 65a and 65b disposed on opposing sides of a channel 126 (Fig. 4C) that is configured to facilitate reciprocation of a cutting mechanism or knife 85 therethrough (Fig. 2), as discussed in detail below.

Each shaft 12a and 12b includes a handle 15 and 17, respectively, disposed at the proximal end 14a and 14b thereof. Each handle 15 and 17 defines a finger hole 15a and 17a, respectively, therethrough for receiving a finger of the user. Handles 15 and 17 facilitate movement of the shafts 12a and 12b relative to one another which, in turn, pivot the jaw members 110 and 120 from an open position wherein the jaw members 110 and 120 are disposed in spaced relation relative to one another to a clamping or closed position wherein the jaw members 110 and 120 cooperate to grasp tissue therebetween.

As best seen in Fig. 2, shaft 12a is constructed from two components, namely, 12a1 and 12a2, that are coupled together to form shaft 12a. Likewise, shaft 12b is constructed from two components, namely, 12b1 and 12b2, that are coupled together to form shaft 12b. In some embodiments, component halves 12a1 and 12a2 and component halves 12b1 and 12b2 are ultrasonically welded together at a plurality of different weld points and/or may be mechanically coupled together by any suitable method including snap-fitting, adhesive, fastened, etc.

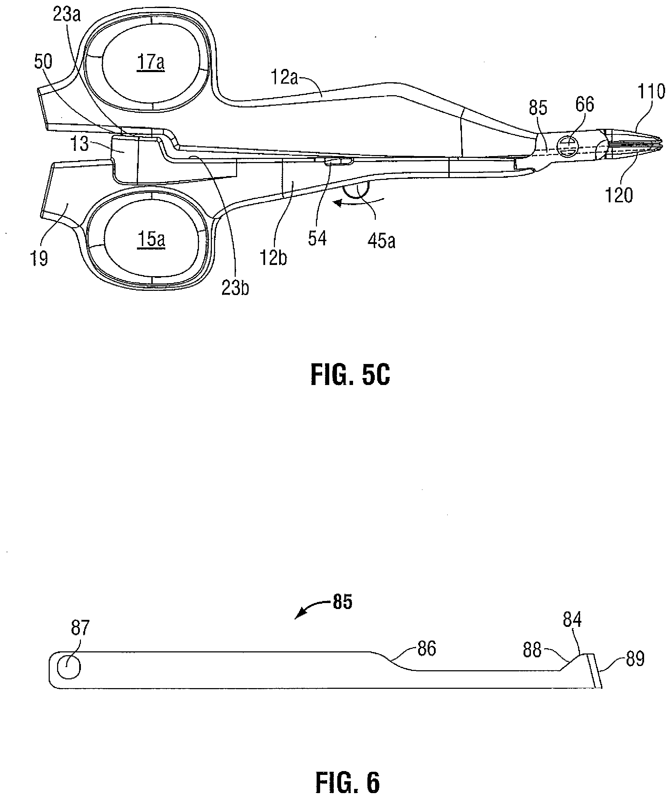

The arrangement of shaft 12b is slightly different from shaft 12a. More particularly, shaft 12a is generally hollow to house the knife 85 and an actuating mechanism 40. The actuating mechanism 40 is operatively associated with a trigger 45 having handle members 45a and 45b disposed on opposing sides of shaft 12a to facilitate left-handed and right-handed operation of trigger 45. Trigger 45 is operatively associated with a series of suitable inter-cooperating elements (e.g., Fig. 2 shows a trigger link 43, a knife pushing link 41, a spring 49, and an anti-deployment link 47) configured to mechanically cooperate (not explicitly shown) to actuate the knife 85 through tissue grasped between jaw members 110 and 120 upon actuation of trigger 45. The spring 49 is coupled between the inner structure of shaft 12b and the trigger link 43. Knife pushing link 41 includes a proximal pivot pin 42a that is received in a friction-fit manner within a pivot aperture 43b defined through the trigger link 43 and a distal pivot pin 42b that is received in a pivot aperture 87 (Fig. 6) defined through a proximal end of knife 85. The trigger 45 is operatively associated with the trigger link 43 such that rotation of the trigger 45 overcomes the biasing force of the spring 49 to rotate the trigger link 43 in a corresponding direction. The trigger link 43 mechanically cooperates with the knife pushing link 41 to reciprocate the knife 85 through knife channel 115 (Fig. 5C). Upon release of the trigger 45, the force of the spring 49 automatically rotates the trigger link 43 counter clock-wise to retract the knife 85 proximally.

The proximal end 14b of shaft 12b includes a switch cavity 13 protruding from an inner facing surface 23b of shaft 12b and configured to seat a depressible switch 50 therein (and the electrical components associated therewith). Switch 50 aligns with an opposing inner facing surface 23a of the proximal end 14a of shaft 12a such that upon approximation of shafts 12a and 12b toward one another, the switch 50 is depressed into biasing engagement with the opposing inner facing surface 23a of the proximal end 14a of shaft 12a.

As shown in Fig. 2, the actuating mechanism 40 includes a cover 53 disposed within the shaft 12a. The cover 53 includes a cantilever spring 52 extending distally from a proximal end thereof. A protrusion 51 is disposed on the distal end of cantilever spring 52 and extends from inner facing surface 23a of shaft 12a toward an opposing pad 54 disposed on inner facing surface 23b of shaft 12b (Fig. 1). Upon approximation of shafts 12a and 12b, protrusion 51 is engaged by pad 54 such that protrusion 51 is biased inward by virtue of the cantilever spring 52 toward shaft 12a to permit further approximation of shafts 12a and 12b and biasing engagement of switch 50 by the opposing inner facing surface 23a of the shaft 12a.

As shown in Fig. 1, an electrosurgical cable 210 having a plug 200 at its proximal end connects the forceps 10 to an electrosurgical generator (not shown). More specifically, the distal end of the cable 210 is securely held to the shaft 12b by a proximal shaft connector 19 and the proximal end of the cable 210 includes a plug 200 having prongs 202a, 202b, and 202c that are configured to electrically and mechanically engage the electrosurgical generator.

The tissue grasping portions of the jaw members 110 and 120 are generally symmetrical and include similar component features that cooperate to permit facile rotation about pivot 65 to effect the grasping and sealing of tissue. As a result, and unless otherwise noted, jaw member 110 and the operative features associated therewith are initially described herein in detail and the similar component features with respect to jaw member 120 will be briefly summarized thereafter.

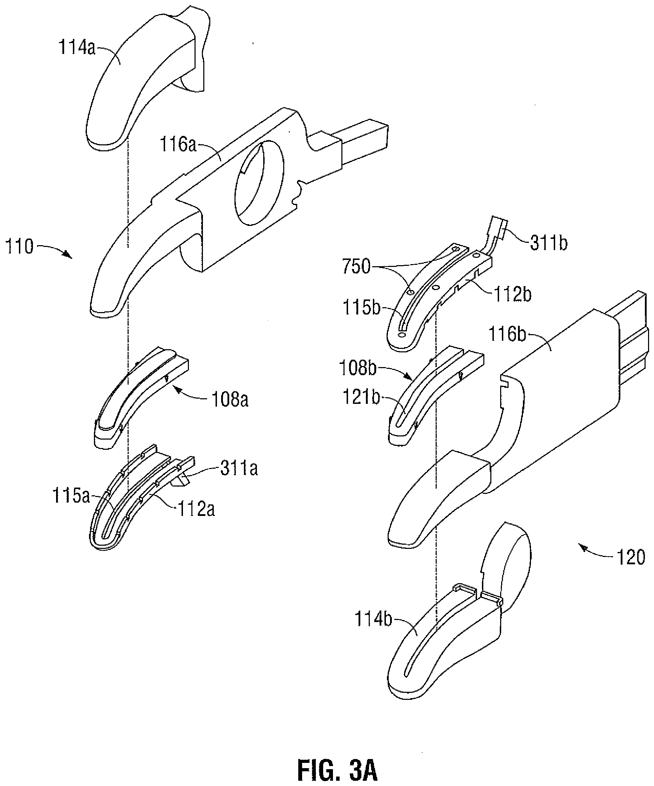

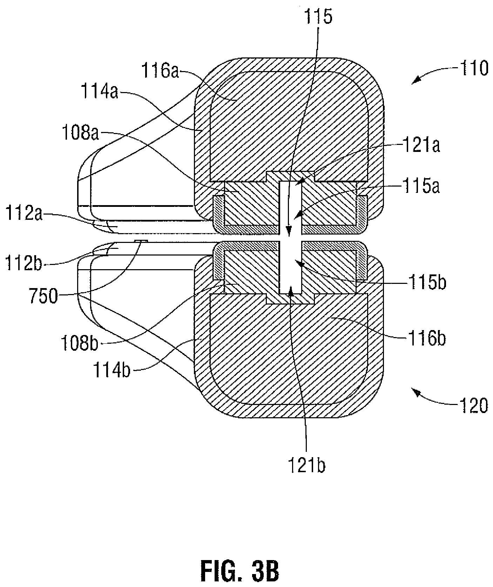

With reference to Figs. 3A and 3B, jaw member 110 includes an outer housing 116a, first and second non-conductive plastic insulators 108a and 114a, and an electrically conductive sealing surface 112a. The first and second insulators 108a and 114a are overmolded about jaw housing 116a in a two-shot overmolding process. More specifically, the first insulator 108a is overmolded about jaw housing 116a to electrically insulate the jaw housing 116a from sealing surface 112a and the second insulator 114a is overmolded about jaw housing 116a to secure the electrically conductive sealing surface 112a thereto. This may be accomplished by stamping, by overmolding, by overmolding a stamped sealing surface, and/or by overmolding a metal injection molded sealing surface. The jaw members 110 and 120 are made from a conductive material. In some embodiments, the jaw members 110 and 120 are powder coated with an insulative coating to reduce stray current concentrations during sealing.

As best shown by the cross-sectional view of Fig. 3B, electrically conductive sealing surface 112a of jaw member 110 is pronounced from the jaw housing 116a and the second insulator 114a such that tissue is grasped by the opposing electrically conductive sealing surfaces 112a and 112b when jaw members 110 and 120 are in the closed position.

Likewise, jaw member 120 includes similar elements that correspond to jaw member 110 including: an outer housing 116b, first and second plastic insulators 108b and 114b, and an electrically conductive sealing surface 112b that is pronounced from the jaw housing 116b and second insulator 114b. As described above with respect to jaw member 110, the first insulator 108b electrically insulates the jaw housing 116b from the sealing surface 112b and the second insulator 114b secures the sealing surface 112b to the jaw housing 116b. Insulators 114a and 114b extend along the entire length of jaw members 110 and 120, respectively, to reduce alternate or stray current paths during sealing. In some embodiments, each of sealing surfaces 112a and 112b may include an outer peripheral edge that has a radius such that each insulator 114a and 114b meets the respective sealing surface 112a and 112b along an adjoining edge that is generally tangential to the radius and/or meets along the radius.

As shown in Figs. 3A and 3B, at least one of the jaw members, e.g., jaw member 120, includes at least one stop member 750 disposed on the inner facing surfaces of the electrically conductive sealing surface 112b and/or 112a. Alternatively or in addition, the stop member(s) 750 may be disposed adjacent to the electrically conductive sealing surfaces 112a, 112b or proximate the pivot 65. The stop member(s) 750 facilitate gripping and manipulation of tissue and to define a gap between opposing jaw members 110 and 120 during sealing and cutting of tissue. In some embodiments, the stop member(s) 750 maintain a gap distance between opposing jaw members 110 and 120 within a range of about 0.001 inches (∼0.03 millimeters) to about 0.006 inches (∼0.015 millimeters).

As shown in Fig. 2, shaft 12b includes a beam 57 disposed therein and extending between handle 15 and jaw member 110. In some embodiments, the beam 57 is constructed of flexible steel to allow the user to generate additional sealing pressure on tissue grasped between the jaw members 110 and 120. More specifically, once end effector assembly 100 is closed about tissue, the shafts 12a and 12b may be squeezed toward each other to utilize the flexibility of the beam 57 to generate the necessary closure pressure between jaw members 110 and 120. In this scenario, the mechanical advantage realized by the compressive force associated with the beam 57 facilitates and assures consistent, uniform, and accurate closure pressure about tissue grasped between jaw members 110 and 120 (e.g., within a working pressure range of about 3 kg/cm² to about 16 kg/cm²). By controlling the intensity, frequency, and duration of the electrosurgical energy applied to the tissue, the user can seal tissue. In some embodiments, the gap distance between opposing sealing surfaces 112a and 112b during sealing ranges from about 0.001 inches to about 0.005 inches.

In some embodiments, the sealing surfaces 112a and 112b are relatively flat to avoid current concentrations at sharp edges and to avoid arcing between high points. In addition, and due to the reaction force of the tissue when engaged, each of jaw members 110 and 120 may be manufactured to resist bending, e.g., tapered along its length to provide a constant pressure for a constant tissue thickness at parallel and the thicker proximal portion of the jaw members 110 and 120 will resist bending due to the reaction force of the tissue.

As shown in Figs. 3A, 3B, 4B, and 4C, at least one of jaw members 110 and 120 includes a knife channel 115a and/or 115b, respectively, disposed therebetween that is configured to allow reciprocation of a knife 85 therethrough. In the illustrated embodiment, a complete knife channel 115 is formed when two opposing channel halves 115a and 115b associated with respective jaw members 110 and 120 come together upon grasping of the tissue. Each plastic insulator 108a and 108b includes a trough 121a and 121b, respectively, that aligns in vertical registration with an opposing knife channel half 115a and 115b, respectively, such that knife 85 does not contact or cut through plastic insulators 108a and 108b upon reciprocation through knife channel 115. In some embodiments, the width of knife channels 115a and 115b and their respective troughs 121a and 121b may be equal along an entire length thereof.

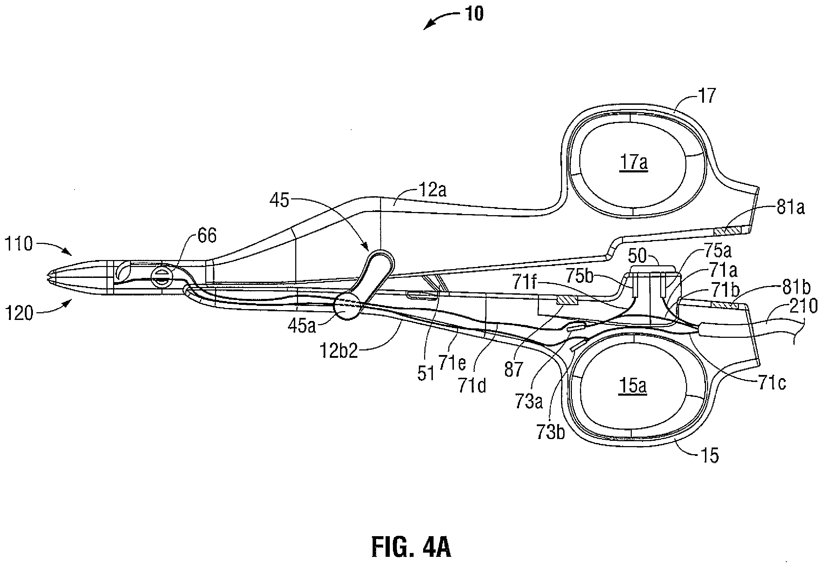

As best shown in Fig. 4A, the interior of cable 210 houses leads 71a, 71b and 71c. Leads 71a, 71b, and 71c extend from the plug 200 through cable 210 and exit the distal end of the cable 210 within the proximal connector 19 of shaft 12b. More specifically, lead 71a is interconnected between prong 202b and a first terminal 75a of the switch 50. Lead 71b is interconnected between prong 202c and a solder sleeve 73a which, in turn, connects lead 71b to an RF lead 71d and to a second terminal 75b of the switch 50 via a connector lead 71f. RF lead 71d carries a first electrical potential of electrosurgical energy from lead 71b to sealing surface 112a. Lead 71c is interconnected between prong 202a and a solder sleeve 73b which, in turn, connects lead 71c to an RF lead 71e. RF lead 71e carries a second electrical potential of electrosurgical energy from lead 71c to sealing surface 112b,

With reference to Fig. 4B, a lead channel 77 is defined in the proximal end of jaw member 110 to provide a pathway for lead 71d to connect to a junction 311a (Fig. 3A) extending from a proximal end of sealing surface 112a. A proximal end of lead channel 77 opens into a raceway 70 that includes a generally elongated configuration with a narrowed proximal end 72 and a broadened distal end 74 that defines an arcuate sidewall 68. Lead 71d is routed to follow a path through the proximal end 72 of raceway 70 and, further, through lead channel 77 for connection to junction 311a.

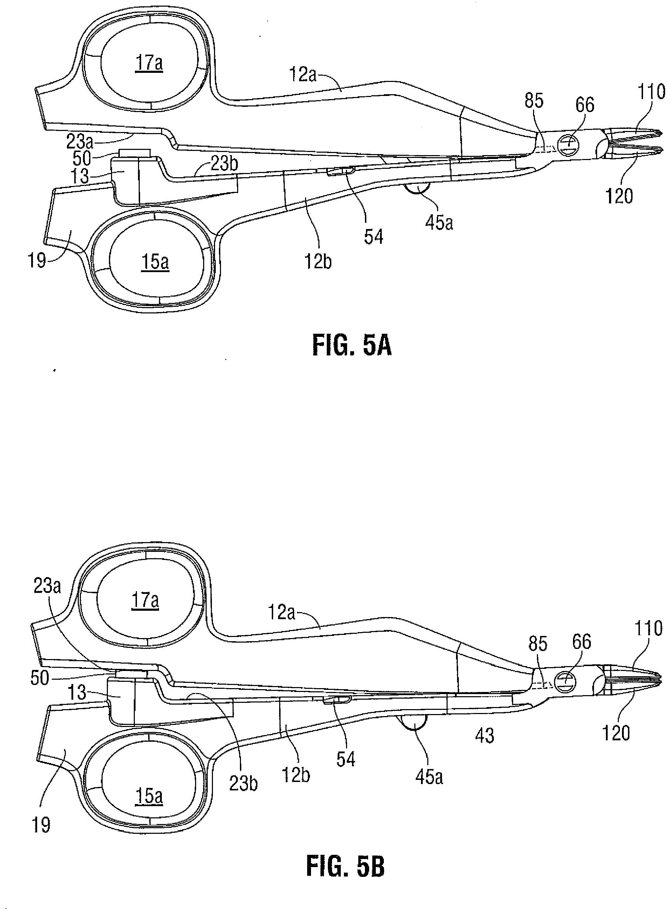

With reference to Fig. 4C, pivot halves 65a and 65b are disposed on opposing sides of channel 126 to facilitate translation of the knife 85 therethrough (Figs. 5A-5C). Pivot halves 65a and 65b are disposed in a split spherical configuration and each include a respective base portion 165a and 165b that support an extension portion 166a and 166b thereon, respectively. Extension portions 166a and 166b are configured to engage correspondingly-dimensioned apertures 67a and 67b, respectively, disposed through pivot plate 66 to pivotably secure jaw member 110 to jaw member 120. A lead channel 109 is defined in the proximal end of jaw member 120 to provide a pathway for lead 71e to connect to a junction 311b extending from a proximal end of sealing surface 112b. Lead 71e is routed to follow a path through raceway 70 and, further between opposing pivot halves 65a and 65b and through lead channel 109 for connection to junction 311b.

With reference to Figs. 5A-5C, as the user applies closure pressure on shafts 12a and 12b to depress switch 50 (Fig. 5B), a first threshold is met corresponding to the closure force applied to switch 50 as a function of displacement of switch 50 that causes switch 50 to generate a first tactile response that corresponds to a complete grasping of tissue disposed between jaw members 110 and 120. Following the first tactile response, as the user applies additional closure pressure on shafts 12a and 12b (Fig. 5C), a second threshold is met corresponding the closure force applied to switch 50 as a function of displacement of switch 50 that causes the switch 50 to generate a second tactile response that corresponds to a signal being generated to the electrosurgical generator to supply electrosurgical energy to the sealing surfaces 112a and 112b. More specifically, the second tactile response indicates closing of a normally open circuit between switch terminals 75a and 75b and, in turn, establishment of an electrical connection between leads 71a and 71b. As a result of the electrical connection between leads 71a and 71b, the electrosurgical generator senses a voltage drop between prongs 202b and 202c and, in response thereto, supplies electrosurgical energy to sealing surfaces 112a and 112b via leads 71d and 71e, respectively.

In one embodiment, the first tactile response indicates to the user that the maximum grasping pressure has been reached before end effector 100 is energized where the user is free to approximate, manipulate, and grasp tissue as needed. In this scenario, the second tactile response indicates to the user the electrosurgical activation of the end effector 100. The switch 50 may include a plurality of other tactile responses between the above discussed first and second tactile responses and/or subsequent to the second tactile response that correspond to particular functions of the forceps 10 such as, for example, operation of the knife 85 and/or the actuation assembly 40, operation of a safety lockout mechanism associated with the actuation assembly 40, as discussed in detail below.

As shown in Fig. 4A, forceps 10 may include a gauge or sensor element 87 disposed within one or both of shafts 12a, 12b such that the clamping or grasping forces being applied to target tissue by end effector 100 may be measured and/or detected. For example, in some embodiments, sensor element 87 may be a strain gauge 87 operably associated with one or both jaw members 110, 120. Sensor element 87 may be one or more Hall effect sensors or strain gauges such as, for example, metallic strain gauges, piezoresistive strain gauges, that may be disposed within one or both of shafts 12a and 12b and/or within one or both of jaw members 110 and 120 to detect tissue pressure. Metallic strain gauges operate on the principle that as the geometry (e.g., length, width, thickness, etc.) of the conductive material changes due to mechanical stress, the resistance of the conductive material changes as a function thereof. This change in resistance is utilized to detect strain or applied mechanical stress such as, for example, the mechanical stress applied to tissue by jaw members 110 and 120. Piezoresistive strain gauges operate based on the changing resistivity of a semiconductor due to the application of mechanical stress.

Hall effect sensors may be incorporated to determine the gap between jaw members 110 and 120 based on a detected relationship between the magnetic field strength between jaw members 110 and 120 and the distance between jaw members 110 and 120.

In some embodiments, one or more reed switches 81a, 81b may be incorporated within shafts 12a and 12b to determine the proximity thereof relative to one another, as shown in Fig. 4A. More specifically, the reed switch(s) may be comprised of a switch 81a disposed within one of the shafts (e.g., shaft 12a) and a magnetic element 81b (e.g., electromagnet, permanent magnet, coil, etc.) disposed within the opposing shaft (e.g., shaft 12a) such that upon approximation of shafts 12a and 12b, the reed switch 81a is activated or closed by the magnetic field of the magnetic element 81b and, likewise, as shafts 12a and 12b are moved away from each other, the lack of magnetic field operates to deactivate or open the reed switch 81a. In this manner, the proximity of shafts 12a and 12b and thus, jaw members 110 and 120, may be determined based on the reaction of the reed switch 81a to the magnetic element 81b.

Any of the above discussed sensors, switches, and/or strain gauge(s) may be incorporated within an electrical circuit such that the strain detected by the strain gauge changes the electrical signal through the circuit. With this purpose in mind, an electrical circuit between the strain gauge and the switch 50 and/or an electrosurgical generator (not shown) allows communication of information such as desired tissue pressure thereto. This information may be tied to the activation of switch 50 such that the switch is not activated until a desired and/or predetermined pressure on tissue grasped between jaw members 110 and 120 is achieved as detected by the strain gauge. Accordingly, the strain gauge may be disposed strategically on the forceps 10, e.g., on one or more of jaw members 110, 120, such that pressure applied to tissue grasped between jaw members 110 and 120 affects the strain gauge.

In use, forceps 10 may be calibrated such that particular tactile responses (e.g., the first tactile response) of switch 50 corresponds to a predetermined grasping pressure on tissue as determined through use of one or more of the above discussed sensors, switches, and/or strain gauge(s). The predetermined grasping pressure about tissue is within the range of about 3 kg/cm² to about 16 kg/cm² in one embodiment and, in another embodiment, about 7 kg/cm² to about 13 kg/cm². In some embodiments, switch 50 may generate multiple tactile responses, each of which corresponds to different predetermined grasping force. For a more detailed discussion of force sensing and/or measuring devices such as load cells, strain gauges, etc., reference is made to commonly-owned U.S. Application No. 11/409,154, filed on April 21,2006.

As shown in Figs. 2, 4B, and 4C, the pivot 65 connects through an aperture 125 defined through jaw member 120 and matingly engages a pivot plate 66 seated within a circumferential lip or flange 78 (Fig. 4B) defined around the periphery of aperture 125 such that the pivot 65 is rotatably movable within the aperture 125 to move jaw members 110 and 120 between open and closed positions.

In some embodiments, actuation of the knife 85 is associated with activation of the switch 50. For example, sensor 87 may be embodied as a position sensor configured to detect the position of knife 85 relative to jaw members 110 and 120 and/or relative to tissue held therebetween. Additionally or alternatively, sensor 87 may be configured to detect either of the first and second tactile responses of switch 50 and allow or prevent actuation of the knife 85 accordingly. For example, based on feedback from the sensor 87, any one or more inter-cooperating elements or lockout mechanisms associated with the actuating mechanism 40 may be energized or deenergized to allow or prevent actuation of the knife 85, as described in more detail below.

As shown in Fig. 6, knife 85 includes a step 86 that reduces the profile of the knife 85 toward a distal end thereof. The distal end of the knife 85 has a step 88 that increases the profile of the knife 85 toward a sharpened distal cutting edge 89. The knife 85 includes a chamfered portion 84 where the sharpened distal cutting edge 89 meets the step 88 to facilitate smooth retraction of knife 85 through the knife channel 15.

In some embodiments, the forceps 10 may include a safety lockout mechanism having a series of suitable inter-cooperating elements (e.g., anti-deployment link 47, trigger link 43) that work together to prevent unintentional firing of the knife 85 when the jaw members 110 and 120 are disposed in the open position. Generally, the anti-deployment link 47 mechanically cooperates with the trigger link 43 to prevent advancement of the knife 85 until the jaw members 110 and 120 are closed about tissue. More specifically, the anti-deployment link 47 includes an engagement tooth 47a formed thereon that is releasably received within a notch portion 43a formed in the trigger link 43 (Fig. 2) when the jaw members 110 and 120 are in the open position such that clock-wise rotation of trigger link 43 is prevented so that the knife 85 can not be advanced distally when the jaw members 110 and 120 are in the open position (Fig. 5A). When the jaw members 110 and 120 are moved to the closed position as illustrated in Fig. 5B, the safety lockout mechanism automatically disengages to allow selective actuation of the knife 85. More particularly, a pin (not shown) disposed on protrusion 51 is received within an aperture 47b defined through anti-deployment link 47 to couple anti-deployment link 47 to protrusion 51. In this way, upon closure of jaw members 110 and 120, the protrusion 51 contacts the pad 54 and is biased inward toward shaft 12b, thereby causing counter clock-wise rotation of protrusion 51 and, in turn, clock-wise rotation of the anti-deployment link 47 such that the engagement tooth 47a is released from engagement with the notch portion 43a. Disengagement of tooth 47a from notch portion 43a allows actuation of the trigger 45 to advance the knife 85 distally through knife channel 15 to cut tissue grasped between jaw members 110 and 120, An example of a safety lockout mechanism for use with forceps 10 is described in commonly-owned U.S. Application Serial No, 12/896,100 entitled "Blade Deployment Mechanisms for Surgical Forceps", filed on October 1, 2010.

In some embodiments, any one or more of the inter-cooperating elements of the safety lockout mechanism (e.g., anti-deployment link 47) may be electrically interconnected to the switch 50 and include suitable electro-mechanical components (e.g., springs, rods, solenoids, etc.) configured to be energized via activation of the switch 50 (e.g., via any one of leads 71a, 71b, 71c, 71d, 71e) to mechanically manipulate the safety lockout mechanism. For example, upon electrical conduction through leads 71d and 71e to energize the end effector 100, the anti-deployment link 47 is energized to cause actuation thereof such that the safety lockout mechanism disengages to allow selective actuation of the knife 85. In this scenario, by way of example, selective actuation of the knife 85 may be prevented until switch 50 has been depressed to generate at least the first tactile response.

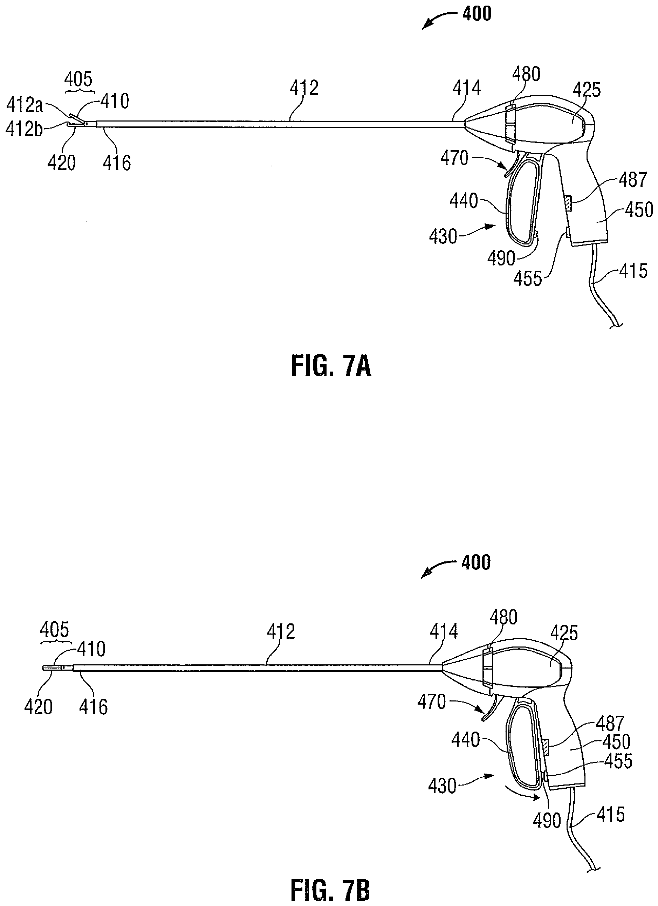

Referring now to Figs. 7A and 7B, a forceps 400 for use with endoscopic surgical procedures is shown in accordance with embodiments of the present disclosure. Forceps 400 operates substantially as described above with respect to forceps 10 (see Figs. 1-6) and will only be discussed to the extent necessary to describe the differences between the embodiments.

Generally, forceps 400 includes a housing 425, a handle assembly 430, a rotating assembly 480, and an end effector assembly 405 that mutually cooperate to grasp, seal, and divide tubular vessels and vascular tissue. The forceps 400 includes a shaft 412 that has a distal end 416 configured to mechanically engage the end effector assembly 405 and a proximal end 414 that mechanically engages the housing 425.

The handle assembly 430 includes a fixed handle 450 and a movable handle 440. Fixed handle 450 is integrally associated with housing 425 and handle 440 is movable relative to fixed handle 450. Rotating assembly 480 is associated with the housing 425 and is rotatable in either direction about a longitudinal axis "A-A". The housing 425 houses the internal working components of the forceps 400.

End effector assembly 405 is coupled to the distal end 416 of shaft 412 and includes a pair of opposing jaw members 410 and 420. Jaw members 410 and 420 each include an electrically conductive tissue sealing surface 412a and 412b, respectively, disposed thereon. Movable handle 440 of handle assembly 430 is operably associated with the end effector assembly 405 through a suitable drive assembly (not shown) to impart movement of the jaw members 410 and 420 from an open position to a clamped or closed position. The drive assembly may be any suitable combination of mechanical, electrical, and/or electromechanical components disposed within the housing 425 and configured to operably associate the movable handle 440 with the end effector assembly 405 such that movement of the movable handle 440 relative to the fixed handle 450 effects movement of the jaw members 410 and 420.

As substantially described above with respect to the jaw members 110 and 120, at least one of the jaw members 410 and 420 includes at least one stop member (not explicitly shown) disposed on the inner facing surfaces of the electrically conductive sealing surface 412b and/or 412a to facilitate gripping and manipulation of tissue and to define a gap between the jaw members 410 and 420 during sealing and cutting of tissue. For example, the stop member(s) may maintain a gap distance between the jaw members 410 and 420 of about 0.001 inches (∼0.03 millimeters) to about 0.006 inches (∼0.015 millimeters).

An electrosurgical cable 415 is coupled at a proximal end to the fixed handle 450 and connects the forceps 400 to an electrosurgical generator (not shown). Electrosurgical cable 415 houses a plurality of electrical leads (e.g., leads 71a, 71b and 71c) that cooperatively operate to supply electrosurgical energy to the electrically conductive sealing surfaces 412a and 412b as substantially described hereinabove with respect to electrosurgical cable 210 of Fig. 1.

Forceps 400 includes a switch 455 disposed on the fixed handle 450 that operates substantially as described above with respect to switch 50 of forceps 10 (see Figs. 1-5C). Although depicted on a lower end of the fixed handle 450 in Figs. 7A and 7B, switch 455 or a similar type switch may be disposed on any suitable location of the fixed handle 450 and, thus, Figs. 7A and 7B are not intended to be limiting with respect to the location of switch 455 on the fixed handle 450.

The lower end of the movable handle 440 includes a switch engaging surface 490 extending therefrom. The switch engaging surface 490 is disposed on a surface of the movable handle 440 that faces the fixed handle 450. When the movable handle 440 is moved toward the fixed handle 450, the switch engaging surface 490 substantially aligns with the switch 455 such that as the moveable handle 440 is moved or squeezed toward the fixed handle 450, as indicated by the rotational arrow depicted in Fig. 7B, the switch 455 is depressed via biasing engagement with the switch engaging surface 490. As the user applies closure pressure on the moveable handle 440 to depress the switch 455 (not explicitly shown), a first threshold is met corresponding to the closure force applied to the switch 455 as a function of displacement of the switch 455 that causes the switch 455 to generate a first tactile response that corresponds to a complete grasping of tissue disposed between jaw members 410 and 420. Following the first tactile response, as the user applies additional closure pressure on the moveable handle 440 (not explicitly shown), a second threshold is met corresponding the closure force applied to the switch 455 as a function of displacement of the switch 455 that causes the switch 455 to generate a second tactile response that corresponds to a signal being generated to the electrosurgical generator to supply electrosurgical energy to the jaw members 410 and 420.

Engagement of the switch 455 by switch engaging surface 490 depresses the switch 455 to meet any one or more thresholds as a function of displacement of switch 455 that, as described above with reference to switch 50 of Fig. 1, causes the switch 455 to generate one or more tactile responses. Each tactile response corresponds to a particular condition. For example, the switch 455 may generate a first tactile response corresponding to a complete grasping of tissue sensed between jaw members 410 and 420 and a second tactile response upon additional depression of the switch 455 relative to the fixed handle 450 corresponding to a signal being generated to the electrosurgical generator to supply electrosurgical energy to the sealing surfaces 412a and 412b.

Although not explicitly shown, at least one of the jaw members 410 and 420 may include a knife channel that is configured to allow reciprocation of a knife therethrough as substantially described above with respect to the knife channel 115a and/or 115b and the knife 85 of Figs. 1-6. Forceps 400 includes a trigger 470 operatively associated with a series of suitable inter-cooperating elements (not explicitly shown) configured to actuate the knife substantially as described above with respect to trigger 45 of Figs. 1-6.

Substantially as described above with respect to the safety lockout mechanism of the forceps 10, forceps 400 may include a safety lockout mechanism having a series of suitable inter-cooperating elements that work together to prevent unintentional firing of the knife when the jaw members 410 and 420 are disposed in the open position. By way of example, the forceps 400 may include inter-cooperating elements such as or substantially similar to the anti-deployment link 47 and trigger link 43 described hereinabove to prevent advancement of the knife until the jaw members 410 and 420 are closed about tissue as substantially described above with respect to the safety lockout mechanism of the forceps 10. An example of a safety lockout mechanism for use with forceps 400 is described in commonly-owned U.S. Application Serial No. 12/896,100 entitled "Blade Deployment Mechanisms for Surgical Forceps," filed on October 1, 2010.

In some embodiments, any one or more of the inter-cooperating elements of the safety lockout mechanism may be electrically interconnected to the switch 455 and include suitable electro-mechanical components (e.g., springs, rods, solenoids, etc.) configured to be energized via activation of the switch 455 to mechanically manipulate the safety lockout mechanism as substantially described above with respect to the switch 50 of the forceps 10.

Substantially as described above with respect to the sensor element 87 of Fig. 4A, forceps 400 may optionally include a gauge or sensor element 487 disposed within either movable handle 440 or fixed handle 450 (depicted within fixed handle 450 in Figs. 7A and 7B for illustrative purposes) such that the clamping or grasping forces being applied to target tissue by end effector 405 may be measured. For example, in some embodiments, the sensor element 487 may be a strain gauge operably associated with one or both jaw members 410, 420.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A bipolar electrosurgical instrument (400), comprising:
a housing (425);
an elongated shaft (412) extending from the housing and having an end effector (405) coupled to a distal end thereof;
a handle assembly (430) operably coupled to the housing and including a movable handle (440) movable relative to a fixed handle (450) for effecting movement of the jaw members relative to one another from a first position wherein the jaw members are disposed in spaced relation relative to one another to a second position wherein the jaw members cooperate to grasp tissue therebetween, each jaw member configured to connect to a source of electrosurgical energy such that the jaw members are configured to selectively conduct energy through tissue held therebetween to effect a tissue seal; and
a switch (455) disposed on the fixed handle and configured to be depressed to a first position and to at least one subsequent position upon biasing engagement with a switch engaging surface (490) disposed on the movable handle, wherein the switch is configured to cause a first tactile response corresponding to a desired pressure on tissue grasped between the jaw members upon movement of the first switch to the first position and the at least one subsequent position is configured to activate the source of electrosurgical energy to supply electrosurgical energy to the jaw members.

2. The bipolar electrosurgical forceps according to claim 1, further comprising:
a knife channel defined along a length of at least one of the jaw members; and
a cutting mechanism configured to reciprocate along the knife channel to cut tissue grasped between the jaw members.

3. The bipolar electrosurgical forceps according to claim 1, further comprising:
an actuator (470) for selectively advancing the cutting mechanism from a first position wherein the cutting mechanism is disposed proximal to tissue grasped between the jaw members to at least one subsequent position wherein the cutting mechanism is disposed distal to tissue grasped between the jaw members.

4. The bipolar electrosurgical forceps according to claim 1, wherein the switch generates the first tactile response upon movement thereof to the first position and a subsequent tactile response upon movement thereof to the at least one subsequent position.

5. The bipolar electrosurgical instrument according to claim 1, wherein the desired pressure is measured by at least one strain gauge (487) disposed within the forceps.

6. The bipolar electrosurgical forceps according to claim 1, wherein the first position of the switch corresponds to an initial closure pressure of the movable handle relative to the fixed handle and the at least one subsequent position of the switch corresponds to a subsequent closure pressure of the movable handle relative to the fixed handle that is greater than the initial closure pressure.

7. The bipolar electrosurgical instrument according to claim 1, further comprising a safety lockout configured to prevent reciprocation of the cutting mechanism when the jaw members are disposed in the first position.

8. A bipolar electrosurgical instrument according to claim 2, wherein each of the jaw members includes an electrically conductive sealing surface and at least one of the jaw members includes at least one non-conductive stop member disposed on the electrically conductive sealing surface configured to control the distance between opposing electrically conductive sealing surfaces when tissue is held therebetween.

9. The bipolar electrosurgical instrument according to claim 1, comprising:
a knife channel defined along a length of at least one of the jaw members;
a cutting mechanism configured to reciprocate along the knife channel to cut tissue grasped between the jaw members;
an actuator for selectively advancing the cutting mechanism from a first position wherein the cutting mechanism is disposed proximal to tissue grasped between the jaw members to at least one subsequent position wherein the cutting mechanism is disposed distal to tissue grasped between the jaw members.

10. The bipolar electrosurgical forceps according to claim 9, wherein the first position of the switch corresponds to an initial closure pressure of the movable handle relative to the fixed handle and the at least one subsequent position of the switch corresponds to a subsequent closure pressure of the movable handle relative to the fixed handle that is greater than the initial closure pressure.

11. The bipolar electrosurgical instrument according to claim 9, further comprising a safety lockout configured to prevent reciprocation of the cutting mechanism when the jaw members are disposed in the first position.

12. The bipolar electrosurgical forceps according to claim 7 or 11, wherein the safety lockout mechanism has a series of suitable inter-cooperating elements including an anti-deployment link (47) and a trigger link (43) that work together to prevent unintentional firing of the cutting mechanism when the jaw members are disposed in the open position, wherein the anti-deployment link (47) mechanically cooperates with the trigger link (43) to prevent advancement of the cutting mechanism until the jaw members are closed about tissue.

13. The bipolar electrosurgical forceps according to claim 12, wherein the anti-deployment link (47) includes an engagement tooth (47a) formed thereon that is releasably received within a notch portion (43a) formed in the trigger link (43) when the jaw members are in the open position such that rotation of the trigger link (43) is prevented so that the cutting mechanism (85) cannot be advanced distally when the jaw members are in the open position, and wherein the safety lockout mechanism is configured for automatically disengaging to allow selective actuation of the cutting mechanism when the jaw members are moved to the closed position,.

## Patentansprüche

1. Bipolares elektrochirurgisches Instrument (400), umfassend:
ein Gehäuse (425);
einen langgestreckten Schaft (412), der sich von dem Gehäuse erstreckt und einen Endeffektor (405) an einem distalen Ende davon gekoppelt hat;
eine Handgriffbaugruppe (430), die betriebsfähig mit dem Gehäuse gekoppelt ist und einen beweglichen Handgriff (440) aufweist, der relativ zu einem festen Handgriff (450) beweglich ist, um eine Bewegung der Klemmbackenelemente relativ zueinander von einer ersten Position, bei der die Klemmbackenelemente in beabstandeter Beziehung relativ zueinander angeordnet sind, in eine zweite Position zu bewirken, bei der die Klemmbackenelemente zusammenwirken, um Gewebe dazwischen zu ergreifen, wobei jedes Klemmbackenelement konfiguriert ist, um sich mit einer Quelle von elektrochirurgischer Energie zu verbinden, so dass die Klemmbackenelemente konfiguriert sind, um wahlweise Energie durch dazwischen gehaltenes Gewebe zu leiten, um eine Gewebeabdichtung zu bewirken; und
einen Schalter (455), der an dem festen Handgriff angeordnet und konfiguriert ist, um in eine erste Position und in zumindest eine darauffolgende Position gedrückt zu werden, bei vorspannendem Eingriff mit einer an dem beweglichen Handgriff angeordneten Schaltereingriffsfläche (490), wobei der Schalter konfiguriert ist, um eine erste taktile Rückmeldung entsprechend eines gewünschten Drucks auf zwischen den Klemmbackenelementen ergriffenem Gewebe bei Bewegung des ersten Schalters in die erste Position zu bewirken, und die zumindest eine darauffolgende Position konfiguriert ist, um die Quelle von elektrochirurgischer Energie zu aktivieren, um elektrochirurgische Energie an die Klemmbackenelemente zu liefern.

2. Bipolare elektrochirurgische Zange nach Anspruch 1, weiter umfassend:
einen Messerkanal, der entlang einer Länge von zumindest einem der Klemmbackenelemente definiert ist; und
einen Schneidmechanismus, der konfiguriert ist, um sich entlang des Messerkanals hin- und her zu bewegen, um zwischen den Klemmbackenelementen ergriffenes Gewebe zu schneiden.

3. Bipolare elektrochirurgische Zange nach Anspruch 1, weiter umfassend:
einen Aktuator (470) zum wahlweise Vorwärtsbewegen des Schneidmechanismus von einer erste Position, bei der der Schneidmechanismus proximal zu zwischen den Klemmbackenelementen ergriffenem Gewebe angeordnet ist, in zumindest eine darauffolgende Position, bei der der Schneidmechanismus distal zu zwischen den Klemmbackenelementen ergriffenem Gewebe angeordnet ist.

4. Bipolare elektrochirurgische Zange nach Anspruch 1, wobei der Schalter die erste taktile Rückmeldung bei einer Bewegung davon in die erste Position und eine darauffolgende taktile Rückmeldung bei einer Bewegung davon in die zumindest eine darauffolgende Position generiert.

5. Bipolares elektrochirurgisches Instrument nach Anspruch 1, wobei der gewünschte Druck durch zumindest einen Dehnungsmessstreifen (487) gemessen wird, der in der Zange angeordnet ist.

6. Bipolare elektrochirurgische Zange nach Anspruch 1, wobei die erste Position des Schalters einem anfänglichen Schließdruck des beweglichen Handgriffs relativ zu dem festen Handgriff entspricht und die zumindest eine darauffolgende Position des Schalters einem darauffolgenden Schließdruck des beweglichen Handgriffs relativ zu dem festen Handgriff entspricht, der größer als der anfängliche Schließdruck ist.

7. Bipolares elektrochirurgisches Instrument nach Anspruch 1, weiter umfassend eine Sicherheitssperre, die konfiguriert ist, um ein Hinundherbewegen des Schneidmechanismus zu verhindern, wenn die Klemmbackenelemente in der ersten Position angeordnet sind.

8. Bipolares elektrochirurgisches Instrument nach Anspruch 2, wobei jedes der Klemmbackenelemente eine elektrisch leitende Abdichtfläche aufweist und zumindest eines der Klemmbackenelemente zumindest ein nicht leitendes Anschlagelement aufweist, das an der elektrisch leitenden Abdichtfläche angeordnet ist, das konfiguriert ist, um den Abstand zwischen gegenüberliegenden elektrisch leitenden Abdichtflächen zu steuern, wenn Gewebe dazwischen gehalten wird.

9. Bipolares elektrochirurgisches Instrument nach Anspruch 1, umfassend:
einen Messerkanal, der entlang einer Länge von zumindest einem der Klemmbackenelemente definiert ist;
einen Schneidmechanismus, der konfiguriert ist, um sich entlang des Messerkanals hin- und her zu bewegen, um zwischen den Klemmbackenelementen ergriffenes Gewebe zu schneiden;
einen Aktuator zum wahlweise Vorwärtsbewegen des Schneidmechanismus von einer ersten Position, bei der der Schneidmechanismus proximal zu zwischen den Klemmbackenelementen ergriffenem Gewebe angeordnet ist, in zumindest eine darauffolgende Position, bei der der Schneidmechanismus distal zu zwischen den Klemmbackenelementen ergriffenem Gewebe angeordnet ist.

10. Bipolare elektrochirurgische Zange nach Anspruch 9, wobei die erste Position des Schalters einem anfänglichen Schließdruck des beweglichen Handgriffs relativ zu dem festen Handgriff entspricht und die zumindest eine darauffolgende Position des Schalters einem darauffolgenden Schließdruck des beweglichen Handgriffs relativ zu dem festen Handgriff entspricht, der größer als der anfängliche Schließdruck ist.

11. Bipolares elektrochirurgisches Instrument nach Anspruch 9, weiter umfassend eine Sicherheitssperre, die konfiguriert ist, um ein Hinundherbewegen des Schneidmechanismus zu verhindern, wenn die Klemmbackenelemente in der ersten Position angeordnet sind.

12. Bipolare elektrochirurgische Zange nach Anspruch 7 oder 11, wobei der Sicherheitssperrenmechanismus eine Reihe von geeigneten untereinander zusammenwirkenden Elementen, einschließlich einer Anti-Einsatz-Verbindung (47) und einer Auslöse-Verbindung (43), hat, die zusammenarbeiten, um unbeabsichtigtes Auslösen des Schneidmechanismus zu verhindern, wenn die Klemmbackenelemente in der offenen Position angeordnet sind, wobei die Anti-Einsatz-Verbindung (47) mit der Auslöse-Verbindung (43) mechanisch zusammenwirkt, um eine Vorwärtsbewegung des Schneidmechanismus zu verhindern, bis die Klemmbackenelemente über Gewebe geschlossen sind.

13. Bipolare elektrochirurgische Zange nach Anspruch 12, wobei die Anti-Einsatz-Verbindung (47) einen daran ausgebildeten Eingriffszahn (47a) aufweist, der in einem in der Auslöse-Verbindung (43) ausgebildeten Kerbenabschnitt (43a) lösbar empfangen ist, wenn die Klemmbackenelemente in der offenen Position sind, so dass eine Drehung der Auslöse-Verbindung (43) verhindert wird, so dass der Schneidmechanismus (85) nicht distal vorwärts bewegt werden kann, wenn die Klemmbackenelemente in der offenen Position sind, und wobei der Sicherheitssperrenmechanismus für automatisches Lösen konfiguriert ist, um eine wahlweise Betätigung des Schneidmechanismus zu erlauben, wenn die Klemmbackenelemente in die geschlossene Position bewegt werden.

## Revendications

1. Instrument électrochirurgical bipolaire (400), comprenant :
un boîtier (425) ;
un arbre allongé (412) s'étendant du boîtier et ayant un effecteur terminal (405) couplé à son extrémité distale ;
un ensemble de poignée (430) couplé en service au boîtier et comprenant une poignée mobile (440) mobile par rapport à une poignée fixe (450) pour effectuer un mouvement des éléments de mors l'un par rapport à l'autre d'une première position dans laquelle les éléments de mors sont disposés en relation espacée l'un par rapport à l'autre et une seconde position dans laquelle les éléments de mors coopèrent pour saisir un tissu qui se trouve entre eux, chaque élément de mors étant configuré pour se connecter à une source d'énergie électrochirurgicale de sorte que les éléments de mors soient configurés pour conduire sélectivement de l'énergie à travers un tissu maintenu entre eux afin d'effectuer un joint tissulaire étanche ; et
un commutateur (455) disposé sur la poignée fixe et configuré pour être pressé dans une première position et dans au moins une position ultérieure lors d'un engagement de sollicitation avec une surface d'engagement de commutateur (490) disposée sur la poignée mobile, dans lequel le commutateur est configuré pour susciter une première réponse tactile correspondant à une pression souhaitée sur le tissu saisi entre les éléments de mors lors du déplacement du premier commutateur dans la première position et la au moins une position ultérieure est configurée pour activer la source d'énergie électrochirurgicale pour fournir de l'énergie électrochirurgicale aux éléments de mors.

2. Forceps électrochirurgical bipolaire selon la revendication 1, comprenant en outre :
un canal de couteau défini sur une longueur d'au moins l'un des éléments de mors ; et
un mécanisme de coupe configuré pour effectuer un mouvement de va-et-vient le long du canal de couteau afin de couper le tissu saisi entre les éléments de mors.

3. Forceps électrochirurgical bipolaire selon la revendication 1, comprenant en outre :
un actionneur (470) pour faire avancer sélectivement le mécanisme de coupe d'une première position dans laquelle le mécanisme de coupe est disposé de manière proximale au tissu saisi entre les éléments de mors à au moins une position ultérieure dans laquelle le mécanisme de coupe est disposé de manière distale au tissu saisi entre les éléments de mors.

4. Forceps électrochirurgical bipolaire selon la revendication 1, dans lequel le commutateur génère la première réponse tactile lors de son déplacement dans la première position et une réponse tactile ultérieure lors de son déplacement dans la au moins une position ultérieure.

5. Instrument électrochirurgical bipolaire selon la revendication 1, dans lequel la pression souhaitée est mesurée par au moins une jauge de contrainte (487) disposée dans le forceps.

6. Forceps électrochirurgical bipolaire selon la revendication 1, dans lequel la première position du commutateur correspond à une pression de fermeture initiale de la poignée mobile par rapport à la poignée fixe et la au moins une position ultérieure du commutateur correspond à une pression de fermeture ultérieure de la poignée mobile par rapport à la poignée fixe qui est supérieure à la pression de fermeture initiale.

7. Instrument électrochirurgical bipolaire selon la revendication 1, comprenant en outre un verrouillage de sécurité configuré pour empêcher le mouvement de va-et-vient du mécanisme de coupe lorsque les éléments de mors sont disposés dans la première position.

8. Instrument électrochirurgical bipolaire selon la revendication 2, dans lequel chacun des éléments de mors comprend une surface d'étanchéité électroconductrice et au moins l'un des éléments de mors comprend au moins un élément d'arrêt non conducteur disposé sur la surface d'étanchéité électroconductrice configuré pour commander la distance entre les surfaces d'étanchéité électroconductrices opposées lorsque le tissu est maintenu entre elles.

9. Instrument électrochirurgical bipolaire selon la revendication 1, comprenant :
un canal de couteau défini sur la longueur d'au moins l'un des éléments de mors ;
un mécanisme de coupe configuré pour effectuer un mouvement de va-et-vient le long du canal de coupe afin de couper le tissu saisi entre les éléments de mors ;
un actionneur pour faire avancer sélectivement le mécanisme de coupe d'une première position dans laquelle le mécanisme de coupe est disposé de manière proximale au tissu saisi entre les éléments de mors à au moins une position ultérieure dans laquelle le mécanisme de coupe est disposé de manière distale au tissu saisi entre les éléments de mors.

10. Forceps électrochirurgical bipolaire selon la revendication 9, dans lequel la première position du commutateur correspond à une pression de fermeture initiale de la poignée mobile par rapport à la poignée fixe et la au moins une position ultérieure du commutateur correspond à une pression de fermeture ultérieure de la poignée mobile par rapport à la poignée fixe qui est supérieure à la pression de fermeture initiale.

11. Instrument électrochirurgical bipolaire selon la revendication 9, comprenant en outre un verrouillage de sécurité configuré pour empêcher le mouvement de va-et-vient du mécanisme de coupe lorsque les éléments de mors sont disposés dans la première position.

12. Forceps électrochirurgical bipolaire selon la revendication 7 ou 11, dans lequel le mécanisme de verrouillage de sécurité a une série d'éléments appropriés coopérant entre eux, comprenant une liaison anti-déploiement (47) et une liaison de déclenchement (43) qui travaillent conjointement pour empêcher une activation non intentionnelle du mécanisme de coupe lorsque les éléments de mors sont disposés en position ouverte, dans lequel la liaison anti-déploiement (47) coopère mécaniquement avec la liaison de déclenchement (43) pour empêcher l'avancement du mécanisme de coupe jusqu'à ce que les éléments de mors soient refermés autour du tissu.

13. Forceps électrochirurgical bipolaire selon la revendication 12, dans lequel la liaison anti-déploiement (47) comprend une dent d'engagement (47a) qui y est formée et qui est reçue de manière libérable dans une partie d'encoche (43a) formée dans la liaison de déclenchement (43) lorsque les éléments de mors sont en position ouverte de sorte que la rotation de la liaison de déclenchement (43) soit empêchée afin que le mécanisme de coupe (85) ne puisse être avancé de manière distale lorsque les éléments de mors sont en position ouverte et dans lequel le mécanisme de verrouillage de sécurité est configuré pour se désengager automatiquement afin de permettre l'actionnement sélectif du mécanisme de coupe lorsque les éléments de mors sont déplacés en position fermée.
